(19) Europäisches Patentamt
European Patent Office
Office européen des brevets

(11) EP 0 915 076 B1

(12) EUROPÄISCHE PATENTSCHRIFT

(45) Veröffentlichungstag und Bekanntmachung des Hinweises auf die Patenterteilung:
**16.01.2002 Patentblatt 2002/03**

(51) Int Cl.[7]: **C07C 45/64**, C07C 49/497, C07B 53/00, B01J 31/18, C07F 15/00

(21) Anmeldenummer: **98120544.6**

(22) Anmeldetag: **30.10.1998**

(54) **Verfahren zur Herstellung von trans-(R,R)-Actinol**

Process for the preparation of trans-(R,R)-actinol

Procédé pour la préparation de trans-(R,R)-actinol

(84) Benannte Vertragsstaaten:
**BE CH DE DK ES FR GB IE IT LI NL SE**

(30) Priorität: **06.11.1997 EP 97119381**
**03.09.1998 EP 98116697**

(43) Veröffentlichungstag der Anmeldung:
**12.05.1999 Patentblatt 1999/19**

(73) Patentinhaber: **F. HOFFMANN-LA ROCHE AG**
**4070 Basel (CH)**

(72) Erfinder:
- **Crameri, Yvo**
  **4104 Oberwil (CH)**
- **Puentener, Kurt**
  **4054 Basel (CH)**
- **Scalone, Michelangelo**
  **4127 Birsfelden (CH)**

(56) Entgegenhaltungen:
**WO-A-97/20789** **US-A- 4 072 715**

- **HASHIGUCHI S ET AL: "Asymmetric Transfer Hydrogenation of Aromatic Ketones Catalyzed by Chiral Ruthenium(II) Complexes" J. AM. CHEM. SOC. (JACSAT,00027863);95; VOL.117 (28); PP.7562-3, XP002092422 Research Development Corporation of Japan;ERATO Molecular Catalysis Project; Toyota; 470-03; Japan (JP)**
- **HAACK K J ET AL: "The catalyst precursor, catalyst and intermediate in the RuII-promoted asymmetric hydrogen transfer between alcohols and ketones" ANGEW. CHEM., INT. ED. ENGL. (ACIEAY,05700833);97; VOL.36 (3); PP.285-288, XP002092423 Res. Development Corporation Japan;ERATO Molecular Catalysis Project; Toyota; 470-03; Japan (JP)**
- **FUJII A ET AL: "Ruthenium(II)-Catalyzed Asymmetric Transfer Hydrogenation of Ketones Using a Formic Acid-Triethylamine Mixture" J. AM. CHEM. SOC. (JACSAT,00027863);96; VOL.118 (10); PP.2521-2, XP002092424 Research Development Corporation of Japan;Molecular Catalysis Project; Toyota; 470-03; Japan (JP)**
- **BRUNNER H ET AL: "Asymmetric catalysis. LXXXII. Enantioselective hydrogenation of 4-oxoisophorone" J. ORGANOMET. CHEM. (JORCAI,0022328X);93; VOL.456 (1); PP.71-5, XP002092425 Institut fuer Anorganische Chemie, Universitaet Regensburg, Universitaetsstrasse 31;Regensburg; W-8400; Germany (DE)**
- **SIMAL F ET AL: "Synthesis and evaluation of ruthenium catalysts containing diamine-based ligands in olefin cyclopropanation" TETRAHEDRON LETT. (TELEAY,00404039);98; VOL.39 (21); PP.3493-3496, XP004117612 University of Liege;Laboratory of Macromolecular Chemistry and Organic Catalysis, C.E.R.M.; Liege; B-4000; Belg. (BE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 915 076 B1

Printed by Jouve, 75001 PARIS (FR)

## Beschreibung

Die vorliegende Erfindung betrifft ein Verfahren zur Herstellung von trans-(R,R)-Actinol durch diastereoselektive Transferhydrierung in Gegenwart eines Amino-Amid-Ruthenium-Komplexes als Katalysator.

Optisch aktives Actinol ist bekannt u.a. als wichtiger Baustein für die Synthese von Carotinoiden, wie beispielsweise von Zeaxanthin [Pure & Appl. Chem. 51, 535-564 (1979)]. Actinol wird in den bekannten Verfahren aus dem Trimethylcyclohexadion Levodion hergestellt. Ein solches Verfahren beruht beispielsweise auf der katalytischen Hydrierung von Levodion in Gegenwart von basenarmem Raney-Nickel [Helv. Chim. Acta 59, 1832 (1976)]. Die schlechte Diastereoselektivität der heterogenen Hydrierung, welche zu einem 3-4 : 1-Gemisch von trans- und cis-Actinol führt, zieht jedoch durch die aufwendige Reinigung einen Ausbeuteverlust nach sich; ausserdem besteht die Gefahr einer Racemisierung des Levodions unter den bei der heterogenen Katalyse verwendeten Reaktionsbedingungen. Eine alternative Reinigung durch eine geeignete Destillierkolonne ist zwar in EP-A 0 775 685 beschrieben, die Ausbeuten sind jedoch bescheiden. Das Interesse an Herstellungsmethoden, welche Actinol mit hoher enantiomerer und diastereomerer Reinheit liefern, ist also nach wie vor gross.

Ueberraschenderweise wurde nun gefunden, dass (R)-Levodion durch Transferhydrierung in Gegenwart eines Amino-Amid-Ruthenium-Komplexes in guter chemischer und in hoher optischer Ausbeute in (R,R)-Actinol übergeführt werden kann.

Die Erfindung betrifft somit ein Verfahren zur Herstellung von trans-(R,R)-Actinol (1)

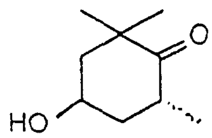

1

durch diastereoselektive Transferhydrierung von Levodion, dadurch gekennzeichnet, dass man (R)-Levodion (2)

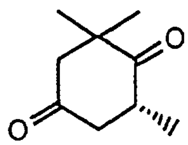

2

in Gegenwart eines Wasserstoffdonors, welcher gleichzeitig als Lösungsmittel verwendet werden kann, und eines Amino-Amid-Ruthenium-Komplexes, wie dieser unten näher definiert wird, hydriert.

Verfahren zur Herstellung von Alkoholen aus Ketonen durch Transferhydrierung sind bekannt. So haben beispielsweise R. Noyori et al. [Acc. Chem. Res. 30, 97-102 (1997)] die asymmetrische Transferhydrierung von Arylalkylketonen untersucht, wie beispielsweise Acetophenon, welche mit hoher optischer und chemischer Ausbeute in Gegenwart eines Wass erstoffdonors, z.B. Isopropanol/Kaliumhydroxid oder Essigsäure/Triethylamin, und eines Rutheniumkomplexes hydriert werden können. Sowohl die chemischen als auch die optischen Ausbeuten verschlechtern sich jedoch beträchtlich, wenn beispielsweise Dialkylketone als Substrate eingesetzt werden.

Für das erfindungsgemässe Verfahren wird ein Amino-Amid-Ruthenium-Komplex der allgemeinen Formel

$$RuH(L\{-H\})(Y) \qquad I$$

eingesetzt, worin

Y einen neutralen Liganden,

L ein gegebenenfalls optisch aktives, monosulfonyliertes Diamin der allgemeinen Formel

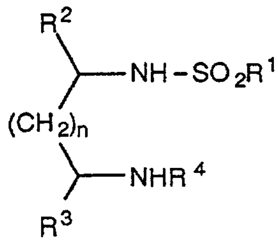

II

R1 gegebenenfalls einfach oder mehrfach fluoriertes Alkyl, Alkenyl, Alkinyl, Cycloalkyl, gegebenenfalls einfach oder mehrfach substituiertes Aryl, Heteroaryl oder Campher-10-yl,

$R^2$ und $R^3$ unabhängig voneinander Wasserstoff, Alkyl, Cycloalkyl oder gegebenenfalls einfach oder mehrfach substituiertes Aryl, oder $R^2$ und $R^3$ zusammen mit der verbindenden Gruppierung $-CH-(CH_2)_n-CH-$ einen Carbocyclus mit 4 bis 8 Kohlenstoffatomen,

$R^4$ Wasserstoff oder Alkyl, und

n 0, 1, 2 oder 3 bedeuten.

Das monosulfonylierte Diamin L liegt im Komplex als Monoanion vor, und dementsprechend wird es in der Formel I als "L{-H}" bezeichnet.

Im Rahmen der vorliegenden Erfindung umfasst der Ausdruck "Alkyl" geradkettige oder verzweigte, gegebenenfalls chirale Alkylgruppen mit 1 bis 8 Kohlenstoffatomen, vorzugsweise mit 1 bis 5 Kohlenstoffatomen. Beispiele solcher Gruppen sind Methyl, Ethyl, Propyl, Isopropyl, n-Butyl, Isobutyl, tert. Butyl, n-Pentyl und Isopentyl. Beispiele der einfach oder mehrfach fluorierten Alkylgruppen sind Trifluormethyl, 2,2,2-Trifluorethyl und Pentafluorethyl.

Der Ausdruck "Alkenyl" umfasst geradkettige oder verzweigte Alkenylgruppen mit 3 bis 8 Kohlenstoffatomen, z.B. Allyl, 2-Butenyl und 3-Butenyl.

Der Ausdruck "Alkinyl" umfasst geradkettige oder verzweigte, eine Dreifachbindung aufweisende Alkinylgruppen mit 3 bis 8 Kohlenstoffatomen, z.B. Propinyl und Butinyl.

Der Ausdruck "Cycloalkyl" bedeutet eine 3- bis 7-gliedrige alicyclische Gruppe, nämlich Cyclopropyl, Cyclobutyl, Cyclopentyl, Cyclohexyl oder Cycloheptyl, von denen Cyclopentyl und Cyclohexyl bevorzugt sind.

Der Ausdruck "gegebenenfalls einfach oder mehrfach substituiertes Aryl" umfasst eine Phenyl- oder Naphthylgruppe, welche unsubstituiert, einfach substituiert oder mehrfach substituiert sein kann. Als Substituenten kommen z.B. in Frage Phenyl, Halogen und geradkettige und verzweigte Alkyl- und Alkoxygruppen mit jeweils 1 bis 5 Kohlenstoffatomen, wobei die mehrfach substituierten Phenyl- oder Naphthylgruppen gleiche oder verschiedene Substituenten aufweisen können. Von den Alkyl- und Alkoxygruppen ist die Methyl- bzw. Methoxygruppe bevorzugt. Beispiele der gegebenenfalls substituierten Arylgruppen sind Phenyl, Chlor-, Brom- und Fluorphenyl, Tolyl, Anisyl, 2,4-Dimethylphenyl sowie Naphthyl.

Der Ausdruck "Heteroaryl" umfasst 5- oder 6-gliedrige 0, S oder N aufweisende heterocyclische Gruppen, wie beispielsweise Furyl, Thienyl, Benzofuryl, Dibenzofuryl, Xanthenyl, Pyrrolyl und Pyridinyl. Besonders bevorzugt sind die O aufweisenden heterocyclischen Gruppen.

Unter dem Ausdruck "neutraler Ligand" ist im Rahmen der vorliegenden Erfindung ein Aren, insbesondere Benzol, Naphthalin oder Tetralin zu verstehen, das im Falle von Benzol einfach oder mehrfach mit geradkettigen oder verzweigten Alkyl- und/oder Alkoxygruppen mit 1 bis 5 Kohlenstoffatomen, vorzugsweise Methyl- bzw. Methoxygruppen, und/oder mit Cycloalkylgruppen substituiert sein kann. Beispiele solcher Liganden sind Benzol, p-Cymol, Toluol, Anisol, Xylol, 1,3,5-Trimethylbenzol, p-Dicyclohexylbenzol, Naphthalin und Tetralin.

Das als Ausgangsmaterial im erfindungsgemässen Verfahren verwendete (R)-Levodion kann bekanntlich u. a. durch Fermentation gewonnen werden und ist zudem im Handel erhältlich.

Eine besondere Klasse von im erfindungsgemässen Verfahren verwendeten Amino-Amid-Ruthenium-Komplexen besteht aus denjenigen der Formel I, welche einen Liganden der Formel II aufweist, worin $R^2$ und $R^3$ unabhängig voneinander Wasserstoff, Alkyl, Cycloalkyl oder gegebenenfalls einfach oder mehrfach substituiertes Aryl, oder $R^2$ und $R^3$ zusammen mit der verbindenden Gruppierung $-CH-(CH_2)_n-CH-$ einen Carbocyclus mit 4 bis 6 Kohlenstoffatomen, und n 0 oder 1 bedeuten, und $R^1$ und $R^4$ die oben angegebenen Bedeutungen besitzen.

Bevorzugte monosulfonylierte Diamin-Liganden L sind diejenigen der Formel II, worin $R^2$ und $R^3$ die gleiche Bedeutung haben und entweder Wasserstoff oder Phenyl bedeuten. Ebenfalls bevorzugt sind die Liganden L, worin $R^2$ und $R^3$ zusammen mit der verbindenden Gruppierung $-CH-(CH_2)_n-CH-$ einen Carbocyclus mit 4 bis 8 Kohlenstoffatomen bilden. In beiden Fällen bedeutet $R^4$ vorzugsweise Wasserstoff. Von diesen bevorzugten Liganden L sind insbesondere diejenigen bevorzugt, worin n 0 bedeutet.

Ganz besonders bevorzugte Liganden L sind die optisch aktiven monosulfonylierten Diamin-Liganden der Formel II, worin sowohl $R^2$ wie auch $R^3$ eine andere Bedeutung als Wasserstoff hat.

Ungeachtet davon, ob die monosulfonylierten Diamin-Liganden L optisch aktiv oder nicht sind, sind diejenigen Liganden L der Formel II bevorzugt, worin $R^1$ eine Tolyl-, Anisyl- oder Naphthylgruppe bedeutet.

Beispiele der ganz bevorzugten monosulfonylierten Diamin-Liganden L sind:

(1S,2S)-N-(2-Amino-1,2-diphenyl-ethyl)-4-methyl-benzolsulfonamid,
(1R,2R)-N-(2-Amino-1,2-diphenyl-ethyl)-4-methyl-benzolsulfonamid,
(1RS, 2RS)-N-(2-Amino-1,2-diphenyl-ethyl)-4-methyl-benzolsulfonamid (racemisch),
(1S,2S)-N-(2-Amino-1,2-diphenyl-ethyl)-4-methoxy-benzolsulfonamid,
Naphthalin-1-sulfonsäure [ (1S,2S)-(2-amino-1,2-diphenyl-ethyl)-amid],
(1R,2R)-N-(2-Amino-cyclohexyl)-4-methyl-benzolsulfonamid,
(1RS,2RS)-N-(2-Amino-cyclohexyl)-4-methyl-benzolsulfonamid (racemisch),
N-(2-Amino-ethyl)-4-methyl-benzolsulfonamid und
N-(3-Amino-propyl)-4-methyl-benzolsulfonamid.

Während die ersten sieben Verbindungen chiral sind, handelt es sich bei der acht- und der neunterwähnten Verbindung um nicht chirale Verbindungen.

Das erfindungsgemässe Verfahren (die Transferhydrierung) erfolgt zweckmässig in einem Lösungsmittel, welches gleichzeitig als Wasserstoffdonor dient, oder in einem Gemisch eines Wasserstoffdonors mit einem geeigneten inerten Lösungsmittel. Als Wasserstoffdonor und zugleich Lösungsmittel können insbesondere Alkohole, vorzugsweise sekundäre Alkohole, z.B. Isopropanol, verwendet werden. Als Gemische von Wasserstoffdonoren mit einem inerten Lösungsmittel werden zweckmässigerweise Gemische von Alkoholen mit inerten Lösungsmitteln, vorzugsweise ein Gemisch eines Alkohols mit einem niederen aliphatischen halogenierten Kohlenwasserstoff, z.B. Methylenchlorid oder Ethylenchlorid, verwendet. Weitere geeignete Wasserstoffdonor/Lösungsmittel-Gemische sind Gemische von Ameisensäure oder einem Salz davon mit einem inerten Lösungsmittel, z.B. Triethylamin. Ganz besonders bevorzugt wird als Wasserstoffdonor/Lösungsmittel Isopropanol verwendet. Dabei wird Aceton gebildet, welches gegebenenfalls kontinuierlich aus dem Reaktionssystem entfernt werden kann. Falls das Reduktionsmittel Ameisensäure oder ein Salz davon in einem inerten Lösungsmittel verwendet wird, setzt man in der Regel eine etwa stoichiometrische Menge der Ameisensäure oder eines Salzes davon bezogen auf die Menge (R)-Levodion ein.

Im Falle der Verwendung von Gemischen von Wasserstoffdonoren mit inerten Lösungsmitteln gelten im Prinzip sämtliche Mischungsverhältnisse.

Das Verhältnis von Katalysator (Amino-Amid-Ruthenium-Komplex) der Formel I zu (R)-Levodion beträgt zweckmässigerweise etwa 1:20 bis etwa 1:10 000 Mol:Mol, vorzugsweise etwa 1:200 bis etwa 1:2000.

Die erfindungsgemässe diastereoselektive Transferhydrierung erfolgt zweckmässigerweise bei einer Temperatur von etwa 0°C bis etwa 100°C, vorzugsweise von etwa 20°C bis etwa 50°C, und gegebenenfalls unter einem Schutzgas, vorzugsweise Argon. Zudem wird in der Regel unter Normaldruck oder unter leichtem Vakuum (um beispielsweise das Entfernen von aus Isopropanol gebildetem Aceton zu erleichtern) umgesetzt.

Zur Isolierung und Reinigung des so hergestellten trans-(R,R)-Actinols können konventionelle Methoden der organischen Chemie verwendet werden, z.B. Destillation und Kristallisation.

Für die erfindungsgemässe, diastereoselektive Transferhydrierung muss der als Katalysator verwendete Amino-Amid-Ruthenium-Komplex der Formel I nicht unbedingt als solcher eingesetzt werden. Es erweist sich nämlich als praktisch, den Katalysator im Reaktionssystem in situ zu generieren, was nach den unten beschriebenen Herstellungsverfahren erfolgt.

Die als Katalysatoren erfindungsgemäss verwendeten Amino-Amid-Ruthenium-Komplexe der Formel I können wie in Schema I aufgezeichnet hergestellt werden:

Schema I

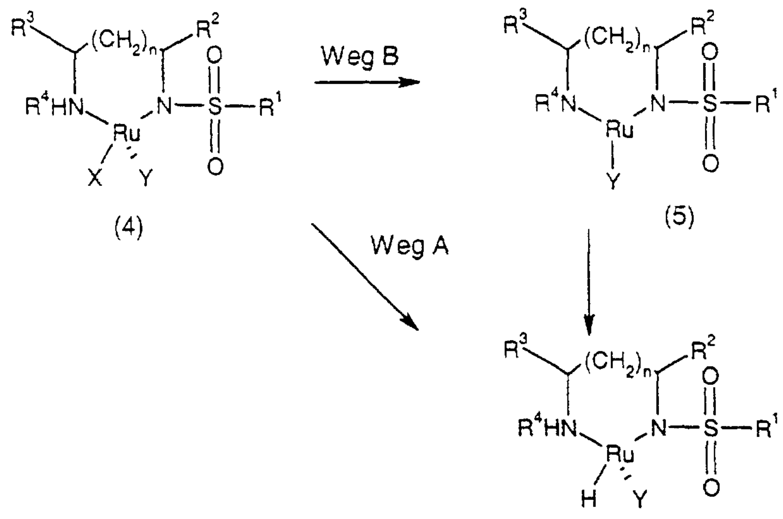


(4) (5)

I

In Schema I haben die Symbole ausser X die obengenannten Bedeutungen; X steht für Chlor, Brom oder Jod, insbesondere für Chlor.

Gemäss Weg A werden die Ruthenium-Katalysatoren der Formel I durch Reaktion des Ruthenium-Halogen-Komplexes des Typs 4 mit einem Hydriddonor, wie beispielsweise Natriumformiat oder Natriummethylat, in einem Lösungsmittel, z.B. in einem niederen Alkohol aber insbesondere im Zweiphasensystem Methylenchlorid/Wasser, generiert. Zweckmässigerweise wird bei Raumtemperatur umgesetzt. Die Komplexbildung ist normalerweise bereits nach etwa 30 Minuten beendet. Diese ist eine an sich bekannte Methode zur Umwandlung von Ru(II)-Halogen- in Ru(II)-Hydrid komplexe [siehe beispielsweise Principles and Applications of Organotransition Metal Chemistry, J.P. Collman et al., University Science Books (1987) und Organometallics 4, 1202 ff. (1985)]. Sie eignet sich zur in situ-Herstellung der Amino-Amid-Ruthenium-Komplexe der Formel I.

Die Ruthenium-Katalysatoren der Formel I können auch durch Umwandlung eines Ruthenium-Halogen-Komplexes des Typs 4 mit einer Base, wie beispielsweise Natriumhydroxid oder Thalliumethylat, in den Ruthenium-Diamid-Komplex des Typs 5 und Weiterreaktion letzteren Komplexes mit einem Alkohol, z.B. dem zugleich als Lösungsmittel agierenden Isopropanol, erhalten werden (Weg B). Als Lösungsmittel können auch in diesem Fall Gemische aus Alkoholen und/oder Wasser mit einem inerten Lösungsmittel, wie beispielsweise Methylenchlorid, eingesetzt werden. Auch in diesem Fall wird zweckmässigerweise bei Raumtemperatur umgesetzt, und die Komplexbildung ist normalerweise innert etwa 30 Minuten beendet.

Weitere Hinweise auf diese an sich bekannten Methoden sind in R. Noyori et al., J. Am. Chem. Soc. 118, 2521 (1996) und R. Noyori et al., Angew. Chemie 109 (3), 297 (1997) beschrieben. In diesen und weiteren Literaturstellen, z.B. in der PCT-Patentpublikation WO 97/20789, sind einige Amino-Amid-Ruthenium-Komplexe der Formel I, worin $R^2$ und $R^3$ jeweils eine andere Bedeutung besitzen als Wasserstoff (welche demzufolge chirale Zentren aufweisen) und deren Herstellung auf obige Weise beschrieben. Die neuen Komplexe dieser Klasse können auf analoge Weise hergestellt werden.

Eine weitere Methode zur Herstellung der als Katalysatoren erfindungsgemässen verwendeten Amino-Amid-Ruthenium-Komplexe der Formel I wird im nachfolgenden Schema II dargestellt:

Schema II: Weg C

$$[RuX_2(Y)]_2 + II + HCOONa + KOH \rightarrow I$$

In diesem Schema bedeutet X Chlor, Brom oder Jod. Y hat die oben angegebene Bedeutung und II steht für das oben angegebene gegebenenfalls optisch aktive, monosulfonylierte Diamin der Formel II (Ligand L). Die Reaktionsbedingungen (Lösungsmittel, Reaktionstemperatur und -dauer) entsprechen denjenigen für Weg A und Weg B.

Die in den oben beschriebenen Komplexen vorhandenen Liganden L (der Formel II) sind zum Teil bekannte Verbindungen [siehe beispielsweise J.A.C.S. 62, 2811-2812 (1940)] und einige davon sind im Handel erhältlich. Optisch aktive Diamine können, falls nicht im Handel erhältlich, durch Spaltung der entsprechenden racemischen Diamine erhalten werden. Die restlichen (neuen) Liganden L können analog den bekannten hergestellt werden.

Besonders bevorzugte Amino-Amid-Ruthenium-Komplexe der Formel I sind diejenigen, in welchen der Ligand L N-(2-Amino-ethyl)-4-methyl-benzolsulfonamid, N-(3-Amino-propyl)-4-methyl-benzolsulfonamid, N-(2-Amino-1,2-diphenyl-ethyl)-4-methyl-benzolsulfonamid oder N-(2-Amino-cyclohexyl)-4-methyl-benzolsulfonamid ist.

Diejenigen Amino-Amid-Ruthenium-Komplexe der Formel I, in welcher L ein monosulfonyliertes Diamin der allgemeinen Formel

$$R^1\ SO^2\text{-HN-}CH_2\text{-}(CH_2)_n\text{-}CH_2\text{-NHR}^4 \qquad II'$$

(d.h. der Formel II, worin $R^2$ und $R^3$ beide Wasserstoff sind) worin $R^1$, $R^4$ und n die oben angegebenen Bedeutungen besitzen, sind neu und ebenfalls Gegenstand der vorliegenden Erfindung. Indem die beiden Kohlenstoffatome, welche die Gruppe NH-$SO_2R^1$ bzw. $NHR^4$ tragen, nicht chiral sind, unterscheiden sich die Diamin-Liganden der Formel II' von denjenigen Diamin-Liganden der Formel II, welche einen Substituenten an diesen Stellen aufweisen ($R^2$ und $R^3$ haben jeweils eine andere Bedeutung als Wasserstoff).

Auch die entsprechenden Amino-Amid-Ruthenium-Komplexe der Formel RuX(L'{-H})(Y), worin X Chlor, Brom oder Jod, L' ein monosulfonyliertes Diamin der Formel II' und Y einen neutralen Liganden bedeuten, sind neu und stellen einen weiteren Aspekt der vorliegenden Erfindung dar.

Die folgenden Beispiele dienen zur Illustrierung der Erfindung und stellen in keiner Weise irgendeine Beschränkung dar. In diesen Beispielen haben die gewählten Abkürzungen folgende Bedeutung en:

| | |
|---|---|
| Ts-DPEN | N-(2-Amino-1,2-diphenyl-ethyl)-4-methyl-benzolsulfonamid |
| (1-NaphthylS)-DPEN | Naphthalin-1-sulfonsäure (2-amino-1,2-diphenyl-ethyl)-amid |
| (p-AnS)-DPEN | N-(2-Amino-1,2-diphenyl-ethyl)-4-methoxy-benzolsulfonamid |
| Ts-1,2-DACH | N-(2-Amino-cyclohexyl)-4-methyl-benzolsulfonamid |
| Ts-en | N-(2-Amino-ethyl)-4-methyl-benzolsulfonamid |
| Ts-pn | N-(3-Amino-propyl)-4-methyl-benzolsulfonamid |
| p-Cym | p-Cymol |
| Me | Methyl |
| Et | Ethyl |
| DC | Dünnschichtchromatographie |
| GC | Kapillar-Gaschromatographie |
| ee | Enantiomeric Excess. |
| de | Diastereomeric Excess |
| RT | Raumtemperatur |

## Biespiel 1

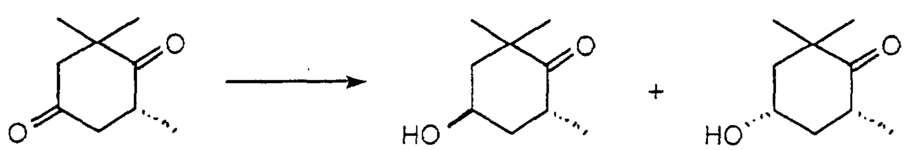

2 1 3

In einer Glove Box ($O_2$-Gehalt < 1ppm) wurden 5,0 g (32,4 mmol) (*R*)-Levodion, 57 ml Isopropanol und 19,5 mg (0,0324 mmol) [Ru((S,S)-Ts-DPEN{-2H})(p-Cym)] in einem 185 ml-Stahlgefäss vorgelegt. Das Stahlgefäss wurde verschlossen und die Transferhydrierung unter Rühren bei 20° C durchgeführt. Innert 24 Stunden war die Hydrierung beendet (> 99% Umsatz). Die Hydrierlösung wurde bei 40°C/50 mbar (5 kPa) eingedampft und der Rückstand im Kugelrohrofen bei 110°C/0,2 mbar (20 Pa) destilliert. Man erhielt 4,9 g eines Gemisches von 94%/5% (*R,R*)-Actinol (**1**)/(*S,R*)- Actinol

(**3**). Der ee-Wert des (*R,R*)-Actinols betrug 99,4%. Die Bestimmung des Umsatzes, des ee- und des de-Wertes erfolgte gaschromatographisch an einer chiralen Phase (BGB-176: 2,3-Dimethyl-6-tert.butyl-dimethyl-silylated-β-cyclodextrin).

**Beispiel 2**

100,0 g (0,648 Mol) (*R*)-Levodion, 1,15 Liter Isopropanol und 0,389 g (0,648 mmol) [Ru((S,S)-Ts-DPEN{-2H}(p-Cym)] wurden in einem 2 L-Vierhalsrundkolben vorgelegt und die Transferhydrierung unter Rühren bei 35°C gestartet. Das bei der Hydrierung entstandene Aceton wurde unter Durchleiten von Argon durch die Reaktionslösung kontinuierlich abgetrennt. Nach 8 Stunden betrug der Umsatz > 99%. Nach Aufarbeitung analog zu Beispiel 1 wurden 98 g eines Gemisches von 94%/6% (*R,R*)-trans-Actinol/(*S,R*)-cis-Actinol erhalten. Der ee-Wert des (*R,R*)-trans-Actinols betrug 99,3%.

**Beispiele 3 -14**

In zu Beispiel 1 analoger Weise wurden die Hydrierungen 3-14 unter den in Tabelle 1 angegebenen Bedingungen durchgeführt:

Tabelle 1

| Bsp. | Aren (Y) | Ligand (L) | T [°C] | t [h] | S/C [m/m] | Ums. [%] | **1/3** | ee **1** [%] |
|---|---|---|---|---|---|---|---|---|
| 3 | p-Cym | (S,S)-Ts-DPEN | 20 | 4 | 200 | > 99 | 95/4 | 99,7 |
| 4 | " | " | 30 | 3 | " | > 99 | 94/5 | 99,8 |
| 5 | " | " | 40 | 2 | " | > 99 | 94/5 | 99,8 |
| 6 | " | " | 50 | 2 | " | > 99 | 93/6 | 99,8 |
| 7 | " | " | 20 | 3 | " | > 99 | 94/4 | 99,6 |
| 8 | " | " | " | 3 | " | > 99 | 94/5 | 99,4 |
| 9 | " | " | " | 3 | " | 98 | 91/6 | 99,1 |
| 10 | " | " | " | 3 | " | 97 | 89/7 | 98,8 |
| 11 | " | " | " | 24 | 500 | 99 | 93/6 | 99,4 |
| 12 | " | " | " | 24 | 1000 | 99 | 94/5 | 99,4 |
| 13 | " | (S,S)-(1-NaphthylS)-DPEN | 40 | 23 | 1000 | 79 | 75/4 | 99,4 |
| 14 | " | (S,S)-(p-AnS)-DPEN | 20 | 23 | 1000 | 96 | 91/6 | 99,3 |
| S/C Substrat/Katalysator (mol/mol)<br>Ums. Umsatz | | | | | | | | |

**Beispiel 15**

Methoden zur *in situ*-Herstellung der Katalysatoren am Beispiel von [RuH(Ts-en{-H})(p-Cym)]1:

Alle Operationen wurden unter Ausschluss von Sauerstoff durchgeführt.

**Methode A (Weg A)**

In 10ml Methylenchlorid und 10ml Wasser wurden 17,73mg (0,26mmol) Natriumformiat und 63,10mg (0,13mmol) [RuCl(Ts-en{-H})(p-Cym)] 15 Minuten bei RT kräftig gerührt. Nach Phasentrennung wurde die organische Phase dreimal mit je 10ml Wasser gewaschen und anschliessend über wasserfreiem Natriumsulfat getrocknet.

**Methode B (Weg A)**

In 3ml Methylenchlorid und 3ml Wasser wurden 5,7mg (0,08mmol) Natriumformiat und 20,2mg (0,04mmol) [RuCl(Ts-en{-H})(p-Cym)] 30 Minuten bei RT kräftig gerührt. Nach anschliessender Phasentrennung wurde die organische Phase über wasserfreiem Natriumsulfat getrocknet. Danach wurde 0,03ml (0,4mmol) Aceton zugesetzt und das Gemisch 15 Minuten gerührt.

**Methode C (Weg B)**

In 7ml Isopropanol wurden 140,70 mg (0,29mmol) [RuCl(Ts-en{-H})(p-Cym)] und 72,5mg (0,29mmol) Thalliumethylat während 30 Minuten bei RT gerührt. Der entstandene Niederschlag wurde abfiltriert.

**Methode D (Weg A)**

In 5ml Methanol wurden 3,88mg (0,06mmol) Natriumformiat und 27,6mg (0,06mmol) [RuCl(Ts-en{-H})(p-Cym)] 30 Minuten bei RT gerührt.

**Methode E (Weg A)**

In 5 ml Methanol wurden 1,12 mg Natriummethylat und 11 mg [RuCl(Ts-en{-H})(p-Cymol)] 30 Minuten bei RT gerührt,

**Beispiel 16**

11,3 g (73,5 mmol) (*R*)-Levodion wurden in einem mit Argon begasten Zweihalsrundkolben in 130 ml Isopropanol suspendiert. Der gemäss Beispiel 15, Methode A aus 71,2 mg (0,147 mmol) [RuCl(Ts-en{-H})(p-Cym)] und 20,0 mg (0,294 mmol) Natriumformiat hergestellte Katalysator wurde unter Luftausschluss zur Levodionsuspension zugegeben. Unter Rühren bei 25°C und kontinuierlichem Abdestillieren des bei der Transferhydrierung entstandenen Acetons war die Reaktion innert 3 Stunden beendet. Der Umsatz betrug 99,8 GC-FI%. Nach Aufarbeitung analog zu Beispiel 1 wurden 10,9 g eines Gemisches von 96%/4% (*R,R*)-Actinol/(*S,R*)-Actinol erhalten. Der ee-Wert des (*R,R*)-Actinols betrug 98,1%.

**Beispiele 17-29**

Die Katalysatorlösungen wurden gemäss den in Beispiel 15 beschriebenen Methoden (Meth.) hergestellt. Die Transferhydrierungen 17-29 wurden analog Beispiel 1 unter den in Tabelle 3 angegebenen Bedingungen durchgeführt:

Tabelle 3

| Bsp. | [RuH(L{-H})(Y)] | | Meth. | t [h] | S/C [m/m] | Ums. [%] | **1/3** | ee **1** [%] |
|---|---|---|---|---|---|---|---|---|
| | L= | Y= | | | | | | |
| 17 | (*1RS,2RS*)-Ts-DPEN | p-Cym | $ | 4 | 200 | 99 | 93/6 | 99,5 |
| 18 | (*R,R*)-Ts-DPEN | p-Cym | $ | 6 | 200 | 98 | 89/7 | 99,7 |
| 19 | (*S,S*)-Ts-DPEN | 1,3,5-$Me_3$-$C_6H_3$ | $ | 4 | 200 | >99 | 92/7 | 99,4 |
| 20 | (*S,S*)-Ts-DPEN | $C_6H_6$ | $ | 4,5 | 200 | >99 | 93/6 | 98,7 |
| 21 | (*S,S*)-Ts-DPEN | p-$Cy_2$-$C_6H_4$ | $ | 24 | 200 | 98 | 92/5 | 94,5 |
| 22 | (*1RS,2RS*)-Ts-1,2-DACH | p-Cym | C | 7 | 200 | >99 | 91/7 | 97,6 |
| 23 | (*1RS,2RS*)-Ts-1,2-DACH | p-Cym | A | 6 | 200 | 91 | 85/6 | 99,1 |
| 24 | (*1RS,2SR*)-Ts-1,2-DACH | p-Cym | A | 16 | 200 | 27 | 25/2 | 99,1 |
| 25 | Ts-en | p-Cym | C | 3 | 200 | >99 | 94/4 | 95,8 |
| 26 | Ts-en | p-Cym | A | 4 | 200 | >99 | 94/4 | 98,8 |
| 27 | Ts-en | p-Cym | D | 5 | 200 | 97 | 91/4 | 85,3 |

$ Der Katalysator wurde hergestellt, isoliert und charakterisiert in Analogie zu R.Noyori et al., Angew. Chem. 109, 297-300 (1997).

Tabelle 3 (fortgesetzt)

| Bsp. | [RuH(L{-H})(Y)] | | Meth. | t [h] | S/C [m/m] | Ums. [%] | **1/3** | ee **1** [%] |
|---|---|---|---|---|---|---|---|---|
| | L= | Y= | | | | | | |
| 28 | Ts-en | p-Cym | B | 16 | 200 | >99 | 94/4 | 99,6 |
| 29 | Ts-pn | p-Cym | A | 22 | 20 | 76 | 68/8 | 96 |
| Alle Beispiele wurden bei Raumtemperatur durchgeführt. Die Substrat-Konzentration betrug 2% (Gewichtsprozent). | | | | | | | | |

**Beispiel 30**

Unter Ausschluss von Sauerstoff wurden in einem 50ml Schlenkrohr 101mg (0,21mmol) [RuCl(Ts-en{-H})(p-Cym)], 1,42g (21,0mmol) Natriumformiat und 0,65g (4,2mmol) (*R*)-Levodion in 21ml Dimethylsulfoxid (DMSO) suspendiert und bei Raumtemperatur 2 Stunden gerührt. Ein schwacher Argonstrom wurde während dieser Zeit durch die Suspension durchgeleitet. Anschliessend wurde das Schlenkrohr verschlossen und die Suspension weitere 14 Stunden gerührt. Danach wurden 10ml Wasser und 50ml Methylenchlorid zugesetzt und die zwei Phasen getrennt. Die wässrige Phase wurde zweimal mit je 50ml Methylenchlorid extrahiert. Die vereinigten organischen Phasen wurden über wasserfreiem Natriumsulfat getrocknet und am Rotationsverdampfer eingedampft. Gemäss GCAnalyse des Rückstandes betrug der Umsatz 99,3% mit einem Gehalt an (*R,R*)-Actinol von 91,2% (76,1%ee).

**Beispiele 31-34**

In zu Beispiel 30 analoger Weise wurden die Hydrierungen 31-34 unter der in Tabelle 4 angegebenen Bedingungen durchgeführt (LM = Lösungsmittel):

Tabelle 4

| Bsp. | [RuH(L{-H})(Y)] | LM | Hydriddonor | C [M] | t [h] | Ums. [%] | **1/3** | ee **1** [%] |
|---|---|---|---|---|---|---|---|---|
| | L= | | | | | | | |
| 31 | (*R,R*)-Ts-DPEN | Methanol | HCOONa | 0,2 | 2 | >99 | 94/6 | 90,5 |
| 32 | (*R,R*)-Ts-DPEN | DMSO | HCOONa | 0,2 | 6 | 85 | 81/4 | 96,2 |
| 33 | Ts-en | Methanol | HCOONa | 0,2 | 3 | >99 | 85/8 | 39,0 |
| 34 | (*R,R*)-Ts-DPEN | HCOOH/$Et_3N$ (5:2) | HCOOH | 1 | 24 | 98 | 85/12 | 81,1 |

**Beispiel 35**

Herstellung der Katalysatorlösung ausgehend von [RuCl(Ts-en{-H})(p-Cym)]

In einen mit Argon begasten Sulfierkolben wurden eine Lösung von 0,662 g (9,73 mmol) Natriumformiat in 120ml Wasser und eine Lösung von 0,942 g (1,95 mmol) [RuCl(Ts-en{-H})(p-Cym)] in 102ml Methylenchlorid zugegeben. Das zweiphasige Gemisch wurde 1 Stunde bei 20-23°C intensiv gerührt. Die Methylenchloridphase enthielt [RuH(Ts-en{-H})(p-Cym)], den aktiven Katalysator.

Transferhydrierung

In einem Sulfierkolben wurden 300,0 g (*R*)-Levodion (1,95 mol) in 3440 ml Isopropanol unter Rühren bei 20-25°C suspendiert, das System mit Argon inertisiert und die Suspension anschliessend auf 40°C aufgeheizt. Die den Katalysator enthaltende Methylenchloridphase wurde unter Ausschluss von Sauerstoff zur Levodionlösung zugegeben. Die Reaktionslösung wurde bei 40°C und einem Teilvakuum von 150 mbar (15 kPa) gerührt, wobei zuerst das Methylenchlorid und dann das bei der Transferhydrierung gebildete Aceton kontinuierlich abdestilliert wurde. Das abdestillierte Aceton/Isopropanol-Gemisch wurde durch Zugabe von frischem Isopropanol ersetzt. Nach 6 Stunden war die Hydrierung beendet. Der Umsatz betrug 99,6% GC-FI%.
Zur Abtrennung des Katalysators wurde die Hydrierlösung eingedampft, der Rückstand in Diisopropylether gelöst und die Lösung mit Aktivkohle behandelt. Nach Filtration und Eindampfen erhielt man 303,2 g eines Gemisches von 95%/5% (*R,R*)-/(*S,R*)-Actinol als Öl. Der ee-Wert des (*R,R*)-Actinols betrug 98,9%. 100 g dieses Gemisches wurden aus

Diisopropylether/n-Hexan kristallisiert. Man erhielt 75 g reines (*R,R*)-Actinol mit einem ee > 99,5%.

**Beispiel 36**

Herstellung der Katalysatorlösung ausgehend von $[RuCl_2(p\text{-}Cym)]_2$ (Weg C)

In einem mit Argon begasten Sulfierkolben wurden 0,199 g (0,324 mmol) $[RuCl_2(p\text{-}Cym)]_2$ und 0,139 g (0,648 mmol) N-(p-Tosyl)-ethylendiamin in 20 ml Methylenchlorid gelöst und eine Lösung von 0,221 g (3,25 mmol) Natriumformiat und 0,036 g (0,64 mmol) Kaliumhydroxid in 20 ml Wasser zugegeben. Das zweiphasige Gemisch wurde 1 Stunde bei 20-23°C intensiv gerührt. Die Methylenchloridphase enthielt [RuH(Ts-en{-H})(p-Cym)], den aktiven Katalysator.

Transferhydrierung

Die Hydrierung von 100,0 g (648,5 mmol) (R)-Levodion erfolgte analog Beispiel 35. Nach 8 Stunden war die Hydrierung beendet. Man erhielt in > 99%iger Ausbeute ein Gemisch von 95%/5% (*R,R*)-Actinol/(*S,R*)-Actinol. Der ee-Wert des (*R,R*)-Actinols betrug 98,9%.

**Beispiel 37**

Herstellung von [RuCl(Ts-en{-H})(p-Cym)]

Unter Argon wurden in einem 2 L-Sulfierkolben 50,0 g (0,233 mol) *N*-(*p*-Tosyl)-ethylendiamin und 71,5 g (0,117 mol) $[RuCl_2(p\text{-}Cym)]_2$ in 650 ml Methylenchlorid, 330 ml Wasser und 230 ml 1M wässrige Kaliumhydroxidlösung gelöst. Das Zweiphasengemisch wurde 30 Minuten bei RT kräftig gerührt. Nach Phasentrennung wurde die wässrige Phase mit 300 ml Methylenchlorid nachextrahiert und die vereinigten organischen Phasen über wasserfreiem Natriumsulfat getrocknet. Nach Filtration und Eindampfen wurde der orangerote Rückstand in 400 ml Hexan digeriert und am Hochvakuum getrocknet. [RuCl(Ts-en{-H})(p-Cym)] wurde als oranger Festkörper in einer Ausbeute von 97 % (114,3 g) isoliert. Eine 10 g-Probe wurde in Methanol kristallisiert, und 8,5 g [RuCl(Ts-en{-H})(p-Cym)] als rote Kristalle wurden isoliert.
$^1$H-NMR (250MHz, $CDCl_3$): 7,75 (d, 2H), 7,17 (d, 2H), 5,7-5,3 (m, 5H), 3,2-2,1 (m br, 6H), 2,34 (s, 3H), 2,12 (s, 3H), 1,22 (d, 6H).
Mikroanalyse: Ber. für $C_{19}H_{27}N_2O_2RuSCl$ (484,02): C 47,15; H 5,62; N 5,79; S 6,62; Cl 7,32; Gef.: C 47,21; H 5,64; N 5,80; S 6,40; Cl 7,23.

**Beispiel 38**

Herstellung von $[RuCl(Ts\text{-}en\{\text{-}H\})(Me_6C_6)]$

Analog Beispiel 37 wurde nach Umsetzung von 97,2 mg (0,4 mmol) *N*-(*p*-Tosyl)-ethylendiamin, 152,4 mg (0,2 mmol) $[RuCl_2(Me_6C_6)]_2$ und 4,6 ml 0,1M wässrige Kaliumhydroxidlösung in Wasser/Methylenchlorid und anschliessender Kristallisation des Rohprodukts in Methanol 106,0 mg (46%) $[RuCl(Ts\text{-}en\{\text{-}H\})(Me_6C_6)]$ als orange Kristalle isoliert.
$^1$H-NMR (250MHz, $CDCl_3$): 7,80 (d, 2H), 7,11 (d, 2H), 3,5-3,2 (br, 2H), 2,5-2,2 (br, 4H), 2,31 (s, 3H), 2,17 (s, 18H).
Mikroanalyse: Ber. für $C_{21}H_{31}N_2O_2RuSCl$ (512,07): C 49,26; H 6,10; N 5,47; S 6,26; Cl 6,92; Gef.: C 49,03; H 5,96; N 5,51; S 6,02; Cl 6,85.

**Beispiel 39**

Herstellung von [RuH(Ts-en{-H})(p-Cym)]

Unter Argon wurden in einem 5ml Schlenkrohr 19,0 mg (0,04 mmol) [RuCl(Ts-en{-H})(p-Cym)] und 26,7 mg (0,4 mmol) Natriumformiat in 2 ml $D_2O$ und 2 ml $CD_2Cl_2$ gelöst und das Zweiphasengemisch 30 Minuten bei RT gerührt. Die gelbe $CD_2Cl_2$-Phase enthielt gemäss $^1$H-NMR-Analyse [RuH(Ts-en{-H})(p-Cym)] und eine kleine Menge unreagierten [RuCl(Ts-en {-H})(p-Cym)].
$^1$H-NMR (250MHz, $CD_2Cl_2$): 7,46 (d, 2H), 7,09 (d, 2H), 5,0-4,5 (m, 4H), 3,0-1,5 (m, 7H), 2,21 (s. 3H), 2,11 (s, 3H), 1,10 (d, 6H), -6,85 (s, 1H).

## Patentansprüche

**1.** Verfahren zur Herstellung von trans-(R,R)-Actinol (1)

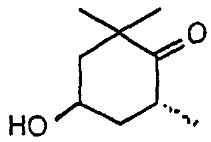

1

durch diastereoselektive Transferhydrierung von Levodion, **dadurch gekennzeichnet, dass** man (R)-Levodion (2)

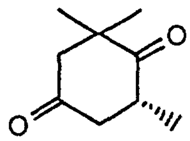

2

in Gegenwart eines Wasserstoffdonors, welcher gleichzeitig als Lösungsmittel verwendet werden kann, und eines Amino-Amid-Ruthenium-Komplexes der allgemeinen Formel

$$RuH(L\{-H\})(Y) \qquad I$$

worin

Y einen neutralen Liganden,
L ein gegebenenfalls optisch aktives, monosulfonyliertes Diamin der allgemeinen Formel

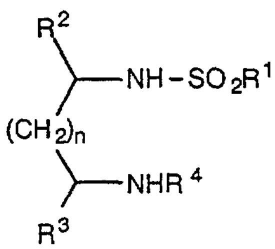

II

$R^1$ gegebenenfalls einfach oder mehrfach fluoriertes Alkyl, Alkenyl, Alkinyl, Cycloalkyl, gegebenenfalls einfach oder mehrfach substituiertes Aryl, Heteroaryl oder Campher-10-yl,
$R^2$ und $R^3$ unabhängig voneinander Wasserstoff, Alkyl, Cycloalkyl oder gegebenenfalls einfach oder mehrfach substituiertes Aryl, oder $R^2$ und $R^3$ zusammen mit der verbindenden Gruppierung $-CH-(CH_2)_n-CH-$ einen Carbocyclus mit 4 bis 8 Kohlenstoffatomen,
$R^4$ Wasserstoff oder Alkyl, und
n 0, 1, 2 oder 3 bedeuten,

hydriert.

**2.** Verfahren nach Anspruch 1, **dadurch gekennzeichnet, dass** in der Formel I des eingesetzten Amino-Amid-Ruthenium-Komplexes L ein gegebenenfalls optisch aktives, monosulfonyliertes Diamin der Formel II bedeutet, worin $R^1$ und $R^4$ die in Anspruch 1 angegebenen Bedeutungen besitzen, $R^2$ und $R^3$ unabhängig voneinander Wasserstoff, Alkyl, Cycloalkyl oder gegebenenfalls einfach oder mehrfach substituiertes Aryl, oder $R^2$ und $R^3$ zusammen mit der verbindenden Gruppierung $-CH-(CH_2)_n-CH-$ einen Carbocyclus mit 4 bis 6 Kohlenstoffatomen, und n 0 oder 1 bedeuten.

**3.** Verfahren nach Anspruch 1 oder 2, **dadurch gekennzeichnet, dass** L ein gegebenenfalls optisch aktives, monosulfonyliertes Diamin der Formel II bedeutet, worin $R^2$ und $R^3$ die gleiche Bedeutung haben und entweder Wasserstoff oder Phenyl bedeuten oder zusammen mit der verbindenden Gruppierung $-CH-(CH_2)_n-CH-$ einen Carbocyclus mit 4 bis 8 Kohlenstoffatomen bilden, und $R^4$ Wasserstoff bedeutet.

**4.** Verfahren nach Anspruch 3, **dadurch gekennzeichnet, dass** in der Gruppierung $-CH-(CH_2)_n-CH-$ n 0 bedeutet.

**5.** Verfahren nach einem der Ansprüche 1 bis 4, **dadurch gekennzeichnet, dass** in der Formel I des eingesetzten Amino-Amid-Ruthenium-Komplexes L ein optisch aktives, monosulfonyliertes Diamin der Formel II bedeutet, worin sowohl $R^2$ wie auch $R^3$ eine andere Bedeutung als Wasserstoff hat.

**6.** Verfahren nach Anspruch 1, **dadurch gekennzeichnet, dass** in der Formel I des eingesetzten Amino-Amid-Ruthenium-Komplexes L ein Ligand ausgewählt aus

(1S,2S)-N-(2-Amino-1,2-diphenyl-ethyl)-4-methyl-benzolsulfonamid,
(R,2R)-N-(2-Amino-1,2-diphenyl-ethyl)-4-methyl-benzolsulfonamid,
(1 RS, 2RS)-N-(2-Amino-1,2-diphenyl-ethyl)-4-methyl-benzolsulfonamid (racemisch),
(1S,2S)-N-(2-Amino-1,2-diphenyl-ethyl)-4-methoxy-benzolsulfonamid,
Naphthalin-1-sulfonsäure [ (1S,2S)-(2-amino-1,2-diphenyl-ethyl)-amid],
(1R,2R)-N-(2-Amino-cyclohexyl)-4-methyl-benzolsulfonamid,
(1RS,2RS)-N-(2-Amino-cyclohexyl)-4-methyl-benzolsulfonamid (racemisch),
N-(2-Amino-ethyl)-4-methyl-benzolsulfonamid und
N-(3-Amino-propyl)-4-methyl-benzolsulfonamid ist.

**7.** Verfahren nach einem der Ansprüche 1 bis 6, **dadurch gekennzeichnet, dass** als Wasserstoffdonor und zugleich Lösungsmittel ein Alkohol, vorzugsweise ein sekundärer Alkohol, insbesondere Isopropanol, verwendet wird.

**8.** Verfahren nach einem der Ansprüche 1 bis 7, **dadurch gekennzeichnet, dass** man als (zusätzliches) Lösungsmittel einen niederen aliphatischen halogenierten Kohlenwasserstoff, z.B. Methylenchlorid oder Ethylenchlorid, oder ein Gemisch von Ameisensäure oder einem Salz davon mit Triethylamin verwendet.

**9.** Verfahren nach einem der Ansprüche 1 bis 8, **dadurch gekennzeichnet, dass** das Verhältnis von Amino-Amid-Ruthenium-Komplex der Formel I zu (R)-Levodion etwa 1:20 bis etwa 1:10000 Mol:Mol, vorzugsweise etwa 1:200 bis etwa 1:2000 Mol:Mol, beträgt.

**10.** Verfahren nach einem der Ansprüche 1 bis 9, **dadurch gekennzeichnet, dass** die diastereoselektive Transferhydrierung bei einer Temperatur von etwa 0°C bis etwa 100°C, vorzugsweise von etwa 20°C bis etwa 50°C, erfolgt.

**11.** Verfahren nach einem der Ansprüche 1 bis 10, **dadurch gekennzeichnet, dass** der als Katalysator verwendete Amino-Amid-Ruthenium-Komplex der Formel I im Reaktionssystem in situ generiert wird.

**12.** Amino-Amid Ruthenium-Komplexe der allgemeinen Formel

$$RuH(L'\{-H\})(Y) \qquad I'$$

worin

Y einen neutralen Liganden,
L' ein monosulfonyliertes Diamin der allgemeinen Formel

$$R^1SO_2\text{-}HN\text{-}CH_2\text{-}(CH_2)_n\text{-}CH_2\text{-}NHR^4 \qquad II'$$

$R^1$ gegebenenfalls einfach oder mehrfach fluoriertes Alkyl, Alkenyl, Alkinyl, Cycloalkyl, gegebenenfalls einfach oder mehrfach substituiertes Aryl, Heteroaryl oder Campher-10-yl,
$R^4$ Wasserstoff oder Alkyl, und

n 0, 1, 2 oder 3 bedeuten.

**13.** Der Amino-Amid-Ruthenium-Komplex nach Anspruch 12, RuH(Ts-en{-H})(p-Cymol).

**14.** Amino-Amid-Ruthenium-Komplexe der allgemeinen Formel

$$RuX(L'\{-H\})(Y) \quad I''$$

worin

X Chlor, Brom oder Jod,
Y einen neutralen Liganden,
L' ein monosulfonyliertes Diamin der allgemeinen Formel

$$R^1SO_2\text{-HN-CH}_2\text{-(CH}_2)_n\text{-CH}_2\text{-NHR}^4 \quad II'$$

$R^1$ gegebenenfalls einfach oder mehrfach fluoriertes Alkyl, Alkenyl, Alkinyl, Cycloalkyl, gegebenenfalls einfach oder mehrfach substituiertes Aryl, Heteroaryl oder Campher-10-yl,
$R^4$ Wasserstoff oder Alkyl, und
n 0, 1, 2 oder 3 bedeuten.

**15.** Der Amino-Amid-Ruthenium-Komplex nach Anspruch 14, RuCl(Ts-en{-H})(p-Cym).

**16.** Der Amino-Amid-Ruthenium-Komplex nach Anspruch 14, RuCl(Ts-en{-H})(Hexamethylbenzol).

**Claims**

**1.** A process for the manufacture of trans-(R,R)-actinol (1)

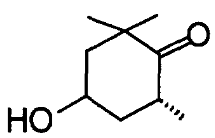

1

by the diasteroselective transfer hydrogenation of levodione, which process comprises hydrogenating (R)-levodione (2)

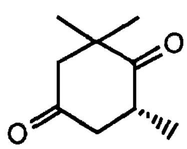

2

in the presence of a hydrogen donor, which can simultaneously be used as the solvent, and an amino-amide-ruthenium complex of the general formula

$$RuH(L\{-H\})(Y) \quad I$$

wherein

Y signifies a neutral ligand,
L signifies an optionally optically active, monosulphonylated diamine of the general formula

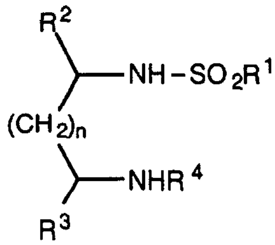

II

$R^1$ signifies optionally mono- or multiply-fluorinated alkyl, alkenyl, alkynyl, cycloalkyl, optionally mono- or multiply-substituted aryl, heteroaryl or camphor-10-yl,
$R^2$ and $R^3$ each independently signify hydrogen, alkyl, cycloalkyl or optionally mono- or multiply-substituted aryl, or $R^2$ and $R^3$ together with the associated grouping $-CH-(CH_2)_n-CH-$ form a carbocycle with 4 to 8 carbon atoms,
$R^4$ signifies hydrogen or alkyl, and
n signifies 0,1,2 or 3.

**2.** A process according to claim 1, wherein in formula I of the employed amino-amide-ruthenium complex L signifies an optionally optically active, monosulphonylated diamine of formula II in which $R^1$ and $R^4$ have the significances given in claim 1, $R^2$ and $R^3$ each independently signify hydrogen, alkyl, cycloalkyl or optionally mono- or multiply-substituted aryl, or $R^2$ and $R^3$ together with the associated grouping $-CH-(CH_2)_n-CH-$ form a carbocycle with 4 to 6 carbon atoms, and n signifies 0 or 1.

**3.** A process according to claim 1 or 2, wherein L signifies an optionally optically active, monosulphonylated diamine of formula II in which $R^2$ and $R^3$ have the same significance and signify either hydrogen or phenyl, or together with the associated grouping $-CH-(CH_2)_n-CH-$ form a carbocycle with 4 to 8 carbon atoms, and $R^4$ signifies hydrogen.

**4.** A process according to claim 3, wherein n in the grouping $-CH-(CH_2)_n-CH-$ signifies 0.

**5.** A process according to any one of claims 1 to 4, wherein in formula I of the employed amino-amide-ruthenium complex L signifies an optically active, monosulphonylated diamine of formula II in which both $R^2$ and $R^3$ have a significance other than hydrogen.

**6.** A process according to claim 1, wherein in formula I of the employed amino-amide-ruthenium complex L is a ligand selected from

(1S,2S)-N-(2-amino-1,2-diphenyl-ethyl)-4-methyl-benzenesulphonamide,
(1R,2R)-N-(2-amino-1,2-diphenyl-ethyl)-4-methyl-benzenesulphonamide,
(1RS,2RS)-N-(2-amino-1,2-diphenyl-ethyl)-4-methyl-benzenesulphonamide (racemic),
(1S,2S)-N-(2-amino-1,2-diphenyl-ethyl)-4-methoxy-benzenesulphonamide,
naphthlene-1-sulphonic acid [ (1S,2S)-(2-amino-1,2-diphenyl-ethyl)-amide],
(1R,2R)-N-(2-amino-cyclohexyl)-4-methyl-benzenesulphonamide,
(1RS,2RS)-N-(2-amino-cyclohexyl)-4-methyl-benzenesulphonamide (racemic),
N-(2-amino-ethyl)-4-methyl-benzenesulphonamide and
N-(3-amino-propyl)-4-methyl-benzenesulphonamide.

**7.** A process according to any one of claims 1 to 6, wherein an alcohol, preferably a secondary alcohol, especially isopropanol, is used as the hydrogen donor and simultaneously as the solvent.

**8.** A process according to any one of claims 1 to 7, wherein a lower aliphatic halogenated hydrocarbon, e.g. methylene chloride or ethylene choride, or a mixture of formic acid of a salt thereof with triethylamine, is used as the (additional) solvent.

**9.** A process according to any one of claims 1 to 8, wherein the ratio of aminoamide-ruthenium complex of formula I to (R)-levodione is about 1:20 to 1:10 000 mol:mol, preferably about 1:200 to about 1:2000 mol:mol.

**10.** A process according to any one of claims 1 to 9, wherein the diastereoselective transfer hydrogenation is effected at a temperature of about 0°C to about 100°C, preferably of about 20°C to about 50°C.

**11.** A process according to any one of claims 1 to 10, wherein the amino-amide-ruthenium complex of formula I used as the catalyst is generated in situ in the reaction system.

**12.** Amino-amide-ruthenium complexes of the general formula

$$RuH(L'\{-H\})(Y) \qquad I'$$

wherein

Y signifies a neutral ligand,
L' signifies a monosulphonylated diamine of the general formula

$$R^1SO_2\text{-HN-}CH_2\text{-}(CH_2)_n\text{-}CH_2\text{-NHR}^4 \qquad II'$$

$R^1$ signifies optionally mono- or multiply fluorinated alkyl, alkenyl, alkynyl, cycloalkyl, optionally mono- or multiply-substituted aryl, heteroaryl or camphor-10-yl,
$R^4$ signifies hydrogen or alkyl, and
n signifies 0, 1, 2 or 3.

**13.** The amino-amide-ruthenium complex according to claim 12, RuH(Ts-en{-H})(p-cymene).

**14.** Amino-amide-ruthenium complexes of the general formula

$$RuX(L'\{-H\})(Y) \qquad I''$$

wherein

X signifies chlorine, bromine or iodine,
Y signifies a neutral ligand,
L' signifies a monosulphonylated diamine of the general formula

$$R^1\ SO_2\text{-HN-}CH_2\text{-}(CH_2)_n\text{-}CH_2\text{-NHR}^4 \qquad II'$$

$R^1$ signifies optionally mono- or multiply-fluorinated alkyl, alkenyl, alkynyl, cycloalkyl, optionally mono- or multiply-substituted aryl, heteroaryl or camphor-10-yl
$R^4$ signifies hydrogen or alkyl, and
n signifies 0, 1, 2 or 3.

**15.** The amino-amide-ruthenium complex according to claim 14, RuCl(Ts-en{-H})(p-Cym).

**16.** The amino-amide-ruthenium complex according to claim 14, RuCl(Ts-en{-H})(hexamethylbenzene).

**Revendications**

**1.** Procédé pour la préparation du *trans*-(R,R)-actinol (1)

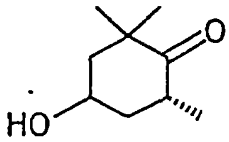

(1)

par hydrogénation diastéréosélective du lévodion par transfert, **caractérisé en ce qu'**on soumet à une hydrogénation du (R)-lévodion (2)

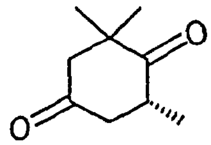

(2)

en présence d'un donneur d'hydrogène, qui peut servir en même temps de solvant, et d'un complexe amino-amide-ruthénium de formule générale

RuH(L{-H})(Y) (I)

dans laquelle

Y représente un ligand neutre,
L représente une diamine monosulfonée, éventuellement optiquement active, de formule générale

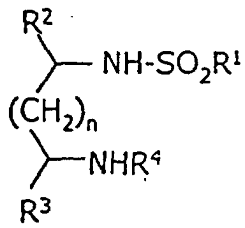

(II)

$R^1$ représente un groupe alkyle éventuellement une ou plusieurs fois fluoré, alcényle, alcynyle, cycloalkyle, un groupe aryle éventuellement une ou plusieurs fois substitué, hétéroaryle, ou camphér-10-yle,
$R^2$ et $R^3$ représentent, indépendamment l'un de l'autre, un atome d'hydrogène ou un groupe alkyle, cycloalkyle ou un groupe aryle éventuellement une ou blusieurs fois substitué, ou $R^2$ et $R^3$ forment ensemble, avec le groupement $-CH-(CH_2)_n-CH-$qui les relie, un groups carbocyclique ayant de 4 à 8 atomes de carbone,
$R^4$ représente un atome d'hydrogène ou un groupe alkyle, et
n représente 0, 1, 2 ou 3.

2. Procédé selon la revendication 1, **caractérisé en ce que**, dans la formule I du complexe amino-amide-ruthénium utilisé, L représente une diamine monosulfonée éventuellement optiquement active de formule II, dans laquelle $R^1$ et $R^4$ ont les significations indiquées dans la revendication 1, $R^2$ et $R^3$ représentent, indépendamment l'un de l'autre, un atome d'hydrogène ou un groupe alkyle, cycloalkyle ou un groupe aryle éventuellement une ou plusieurs fois substitué, ou $R^2$ et $R^3$ forment ensemble, avec le groupement $-CH-(CH_2)_n-CH-$ qui les relie, un groupe carbocyclique ayant de 4 à 6 atomes de carbone, et n est 0 ou 1.

3. Procédé selon la revendication 1 ou 2, **caractérisé en ce que** L représente une diamine monosulfonée éventuellement optiquement active de formule II, dans laquelle $R^2$ et $R^3$ ont la même signification et soit représentent un atome d'hydrogène ou le groupe phényle, soit forment ensemble, avec le groupement $-CH-(CH_2)_n-CH-$ qui les relie, un groupe carbocyclique ayant de 4 à 8 atomes de carbone, et $R^4$ représente un atome d'hydrogène.

**4.** Procédé selon la revendication 3, **caractérisé en ce que** dans le groupement $-CH-(CH_2)_n-CH-$, n est 0.

**5.** Procédé selon l'une quelconque des revendications 1 à 4, **caractérisé en ce que** dans la formule I du complexe amino-amide-ruthénium utilisé, L représente une diamine monosulfonée optiquement active de formule II, dans laquelle aussi bien $R^2$ que $R^3$ ont une autre signification qu'un atome d'hydrogène.

**6.** Procédé selon la revendication 1, **caractérisé en ce que** dans la formule I du complexe amino-amide-ruthénium utilisé, L représente un ligand choisi parmi

le (1S,2S)-N-(2-amino-1,2-diphényléthyl)-4-méthylbenzènesulfonamide,
le (1R,2R)-N-(2-amino-1,2-diphényléthyl)-4-méthylbenzènesulfonamide,
le (1RS,2RS)-N-(2-amine-1,2-diphényléthyl)-4-méthylbenzènesulfonamide (racémique),
le (1S,2S)-N-(2-amine-1,2-diphényléthyl)-4-méthoxybenzènesulfonamide,
le (1S,2S)-(2-amino-1,2-diphényléthyl)amide d'acide naphtalène-1-sulfonique,
le (1R,2R)-N-(2-amino-cyclohexyl)-4-méthylbenzènesulfonamide,
le (1RS,2RS)-N-(2-amino-cyclohexyl)-4-méthylbenzènesulfonamide (racémique),
le N-(2-aminoéthyl)-4-méthylbenzènesulfonamide et
le N-(3-aminopropyl)-4-méthylbenzènesulfonamide.

**7.** Procédé selon l'une quelconque des revendications 1 à 6, **caractérisé en ce qu'**on utilise comme donneur d'hydrogène et en même temps solvant un alcool, de préférence un alcool secondaire, en particulier l'isopropanol.

**8.** Procédé selon l'une quelconque des revendications 1 à 7, **caractérisé en ce qu'**on utilise comme solvant (supplémentaire) un hydrocarbure halogéné aliphatique inférieur, par exemple, le chlorure de méthylène ou le chlorure d'éthylène, ou un mélange d'acide formique, ou d'un sel de celui-ci, avec la triéthylamine.

**9.** Procédé selon l'une quelconque des revendications 1 à 8, **caractérisé en ce que** le rapport du complexe amino-amide-ruthénium de formule I au (R)-lévodion va d'environ 1:20 à environ 1 : 10 000 mole/moles, de préférence, d'environ 1:200 à environ 1 : 2 000 mole/moles.

**10.** Procédé selon l'une quelconque des revendications 1 à 9, **caractérisé en ce que** l'hydrogénation diastéréosélective par transfert s'effectue à une température d'environ 0°C à environ 100°C, de préférence d'environ 20°C à environ 50°C.

**11.** Procédé selon l'une quelconque des revendications 1 à 10, **caractérisé en ce que** le complexe amino-amide-ruthénium de formule I, utilisé en tant que catalyseur, est engendré in situ dans le système réactionnel.

**12.** Complexes amino-amide-ruthénium de formule générale

$$RuH(L'\{-H\})(Y) \qquad (I')$$

dans laquelle

Y représente un ligand neutre,
L représente une diamine monosulfonée de formule générale

$$R'SO_2-NH-CH_2-(CH_2)_n-CH_2-NHR^4 \qquad (II')$$

$R^1$ représente un groupe alkyle éventuellement une ou plusieurs fois fluoré, alcényle, alcynyle, cycloalkyle, un groupe aryle éventuellement une ou plusieurs fois substitué, hétéroaryle, ou camphér-10-yle,
$R^4$ représente un atome d'hydrogène ou un groupe alkyle, et
n représente 0, 1, 2 ou 3.

**13.** Le complexe amino-amide-ruthénium selon la revendication 12 RuH(Ts-ène{-H})(p-cymène).

**14.** Complexes amino-amide-ruthénium de formule générale

$$RuX(L'\{-H\})(Y) \qquad (I'')$$

dans laquelle

X représente le chlore, le brome ou l'iode,
Y représente un ligand neutre,
L représente une diamine monosulfonée de formule générale

$$R'SO_2\text{-}NH\text{-}CH_2\text{-}(CH_2)_n\text{-}CH_2\text{-}NHR^4 \qquad (II')$$

$R^1$ représente un groupe alkyle éventuellement une ou plusieurs fois fluoré, alcényle, alcynyle, cycloalkyle, un groupe aryle éventuellement une ou plusieurs fois substitué, hétéroaryle, ou camphér-10-yle,
$R^4$ représente un atome d'hydrogène ou un groupe alkyle, et
n représente 0, 1, 2 ou 3.

**15.** Le complexe amino-amide-ruthénium selon la revendication 14 RuCl(Ts-ène{-H})(p-cymène).

**16.** Le complexe amino-amide-ruthénium selon la revendication 14 RuCl(Ts-ène{-H})(hexaméthylbenzène).